# EUROPÄISCHE PATENTSCHRIFT

(11) **EP 1 865 885 B1**
(45) Veröffentlichungstag und Bekanntmachung des Hinweises auf die Patenterteilung: **14.04.2010**
(21) Anmeldenummer: 06791356.6
(22) Anmeldetag: 06.09.2006
(51) Int. Cl.: A61F 2/16

(54) **IMPLANTATIONSWERKZEUG FÜR INTRAOKULARLINSEN**
IMPLANTATION TOOL FOR INTRAOCULAR LENSES
OUTIL D'IMPLANTATION DE LENTILLES INTRA-OCULAIRES

(30) Priorität: 09.09.2005 DE 102005042849
(43) Veröffentlichungstag der Anmeldung: 19.12.2007
(73) Patentinhaber: Wollenhaupt, Peter, 59379 Selm-Cappenbaerg (DE); Kammann, Jochen, 44229 Dortmund (DE); Merten, Gerhard, 44534 Lünen (DE)
(72) Erfinder: Wollenhaupt, Peter, 59379 Selm-Cappenbaerg (DE); Kammann, Jochen, 44229 Dortmund (DE); Merten, Gerhard, 44534 Lünen (DE)
(86) Internationale Anmeldenummer: PCT/DE2006/001557
(87) Internationale Veröffentlichungsnummer: WO 2007/028368

(56) Entgegenhaltungen:
- EP-A2- 0 937 443
- WO-A-03/045285

## Beschreibung

Die Erfindung betrifft ein chirurgisches Werkzeug für die Implantation einer Intraokularlinse, kurz IOL genannt, in das menschliche Auge.

### Hintergrund der Erfindung:

IOLs werden benutzt, um die natürliche Augenlinse zu ersetzen oder die Brechkraft zu korrigieren unter Beibehaltung der natürlichen Linse und bestehen aus der künstlichen Optik und der so genannten Haptik. Die Haptik bildet das Tragelement zum Positionieren der IOLs im Kapselsack sulcus ciliaris und der vorderen Augenkammer des Auges. Die IOLs bestehen vorzugsweise aus weichem faltbarem Silikon, Acryl oder Hydrogel und können verschiedenartig konstruiert sein. Aufgrund dieser Materialien ist es möglich, derartige weiche IOLs zu falten oder zu rollen und durch einen kleinen Einschnitt im Auge, der nicht größer als 1-3 mm sein soll, in das Auge einzusetzen. Dieser kleine Einschnitt dient zur Operation von grauem Star, wobei durch phakoemulgierende Aufarbeitung und Absaugung die natürliche Linse durch eine Ultraschall aktivierte spezielle Nadel zerbrochen und das Fragment durch eine kleine Kanüle abgesaugt wird.

Es sind schon verschiedene Verfahren und Vorrichten zum Falten und Rollen sowie des Vorschubs der IOLs angewandt worden, die alle eine Beschädigung der Optik und/oder der Haptik zu vermeiden versuchen. Stellvertretend wird auf einen Stand der Technik hingewiesen, wie er durch
EP 962195 B1, EP 966238 B1 und DE 40 30 492 C1 bekannt ist und Implantationswerkzeuge mit vorgeschalteten Kanülen offenbart, die mit den entsprechend der Sehschwäche angepassten IOLs bestückt werden. Die Implantationswerkzeuge sind zylinderförmig ausgestaltet und werden mit axial beaufschlagtem Kolben mit Kolbenstange und vorgelagertem weichen Stößel bestückt. Mittels der Stößel werden die IOLs aus der Kanüle in das Auge transportiert. Die Betätigung der Kolbenstangen wird in aller Regel vom Operateur per Fingerdruck vorgenommen; es sind jedoch auch manuelle und motorgetriebene Drehantriebe zur Anwendung gekommen. Besonders die motorgetriebene Version des Drehantriebes hatte sich zur Aufgabe gestellt, eine konstante Vortriebsgeschwindigkeit, ein exaktes Einsetzen und eine schonende Behandlung der IOL zu erzielen.

Bei der Handhabung der bekannten Implantationswerkzeuge hat es sich als nachteilig herausgestellt, dass die verhältnismäßig lange Auslegung dieser Werkzeuge dem Operateur ungünstige Hebelverhältnisse beschert, die im Einschnittbereich des Auges zu verhältnismäßig großen unerwünschten und die Operation erschwerenden Bewegungen führt. Durch US 4,699,140 ist ein Implantationswerkzeug bekannt, bei dem die axiale Bewegung der Kolbenstange durch eine radiale Bewegung eines mit einem Exzenter ausgerüsteten Hebels erzielt wird. Eine gleichmäßige Transportbewegung der zu implantierenden IOL kann hier offensichtlich nicht gewährleistet werden.

Durch US 4,976,716 ist darüber hinaus ein Implantationswerkzeug bekannt, das ebenfalls über eine radiale Bewegung eines Hebels den Vortrieb der Kolbenstange bewerkstelligen will, hier soll die radiale Bewegung über ein Ratschengetriebe in eine axiale Bewegung umgelenkt werden. Eine gleichmäßige Bewegung kann hiermit keinesfalls erreicht werden.

Ein hydraulisches Implantationswertzeug ist auch aus der WO-A-03/045285 bekannt.

### Aufgabe der Erfindung:

Die Erfindung will ein Implantationswerkzeug schaffen, das einen gleichmäßigen schonenden Vorschub der IOL, eine kompakte Bauweise und eine Verkürzung des Operationszyklus ermöglicht.

Die erfindungsgemäße Aufgabe wird mit den Merkmalen des Anspruchs 1 gelöst. Bevorzugte Ausführungsformen und vorteilhafte Lösungen ergeben sich aus den Unteransprüchen und der Beschreibung. Erfindungsgemäß wird ein chirurgisches Implantationswerkzeug zum sterilen Implantieren einer verformbaren IOL aus weichem faltbaren Material, insbesondere Silikon, Acryl oder Hydrogel, in ein Auge, geschaffen. Dieses Werkzeug ist mit einem röhrchenförmigen Grundkörper, mit einem röhrcheniormigen im Innendurchmesser an die Linse angepassten Implantierwerkzeug, mit dem die Linse durch eine Schnittöffnung im Auge in die Linsenkapsel des Auges einsetzbar ist, mit einem Stößel und mit einer Schubeinrichtung zum Herausschieben der Linse aus dem Implantierwerkzeug mittels des Stößels versehen. Erfindungsgemäß ist die Schubeinrichtung als Antrieb für den Stößel mit einer Zweistufenhydraulik ausgerüstet bzw. mittels einer Zweistufenhydraulik angetrieben.

### Vorteile der Erfindung:

Der Vorteil der erfindungsgemäßen Lösung wird darin gesehen, dass die IOL mit minimalem Kraftaufwand gleichmäßig, kontrolliert und lagegenau in die Linsenkapsel des Auges oder sonstige gewünschte Orte transportiert und dank eines Rollgangs im Kanülenbereich ein besonders schonender Vorschub bei kleinstmöglichem zusammengerollten Maß erzielt werden kann. Der entscheidende Vorteil stellt sich dadurch ein, dass der Implantationsvorgang präzisiert und beschleunigt wird. Des weiteren kann die IOL in sterilem Zustand bereits vorgeladen sein oder hinzugefügt werden.

### Erläuterung der Erfindung:

Die Erfindung will einen gleichmäßigen Vorschub von Intraokularlinsen durch eine kompakte Bauweise des Implantationswerkzeugs und einen kurzen Operationszyklus schaffen, in dem die Schubstange im Grundkörper des Implantationswerkzeugs durch eine Zweistufenhydraulik angetrieben wird und vorzugsweise eine vorschaltbare Kanüle mit Rollgang den sterilen Aufbewahrungsbehälter für die Intraokularlinse bildet und deren schonenden Transport in das implantationsbereite Auge übernimmt, mithin sogleich das Implantierwerkzeug bildet.

Das Implantationswerkzeug ist vorrangig aufgeteilt in einen Grundkörper und einer Kanüle. Die Kanüle wird durch eine einfache, vorzugsweise lösbare Verbindung, z.B. einen Bajonettverschluss oder eine T-Führung mit Einstelleinrastung, mit dem Grundkörper verbunden. Die Kanüle bildet einen Aufbewahrungsbehälter und gleichzeitig eine Transport-Rollbahn für die IOL. Die IOL mit ihrer Haptik ist in einer sterilen Flüssigkeit in der Kanüle eingelagert. Die Kanüle ist vorderseitig durch eine abziehbare Kappe verschlossen. Die sterile Flüssigkeit wird durch ein einzubringendes Gleitmittel für die IOL ersetzt. Dieses Gleitmittel dient gleichzeitig auch zur Stabilisierung der verschiedenen Raumkompartimente. Die Rückseite der Kanüle ist mit einer Folie verschlossen. Im Zentrum der Kanüle wird die Folie durch den mit einem flexiblen vorderen Ende versehenen Stößel durchfasst und ist in Vorschubkontakt mit der IOL. Durch die axiale Betätigung des kolbennahen Stößelabschnitts des Stößels, der um ein vorgegebenes Anschlagmaß aus dem Grundkörper vorsteht, wird die IOL bis kurz vor dem Austritt aus der Kanüle durch den Rollgang transportiert. Die bei dieser Bewegung vom Kolben verdrängte Flüssigkeit wird über ein entsperrbares Rückschlagventil sowohl in den rückwärtigen Kolbenringraum als auch in den radialen Kolbenringraum der zweiten Hydraulikstufe gedrückt.

Da es sich in der Vergangenheit gezeigt hat, dass der Vortrieb der IOL durch die verwendeten Stößel mit ihrem flexiblen Ende immer wieder zu Beschädigungen der Linse führte, wird nach einem weiteren Merkmal der Erfindung eine Lösung angestrebt, die einen problemlosen Vortrieb der IOL gewährleistet. Hierzu wird dem Implantationswerkzeug eine Gleitmittel beinhaltende Kolbenzylindereinheit zugeordnet, die mit zwei Befüllungsschläuchen bestückt ist. Durch wechselseitiges Betätigen der mit zwei Kolben bestückten Kolbenstange und des Anschlusses der Befüllungsschläuche einerseits an der Kanülenspitze und andererseits am Kanülenende wird ein Austausch der sterilen Aufbewahrungsflüssigkeit kurz vor der Operation durch das Gleitmittel erzielt. Dieser Fiüssigkeitsaustausch ist derart abgestimmt, dass zu erst die Aufbewahrungsflüssigkeit vom Gleitmittel aus dem Kanülenende herausgedrückt wird und anschließend das eingedrückte Gleitmittel zur Kanülenspitze wieder abgesaugt und gleichzeitig am Kanülenende Gleitmittel hineingedrückt wird. Dieser Vorgang läuft synchron mit dem Einfahren des Stößels des Implantationswerkzeuges ab. Bei diesem Schubvorgang entsteht vor der IOL ein hydrostatischer Unterdruck und hinter der IOL ein hydrostatischer Überdruck; somit wird ohne nennenswerte Druckbelastung der IOL durch den Stößel des Implantationswerkzeugs die IOL in ihre gerollte Austrittsposition befördert. Lediglich im völlig zusammengerollten, widerstandsfähigsten Zustand wird die IOL vom Stößel durch die Kanüle in das Auge transportiert. Hierzu bedarfes lediglich einer radialen Betätigung der zweiten Hydraulikstufe durch den Operateur.

Der Operateur benötigt nach dem Entfernen der natürlichen Linse durch den vorher getätigten maximal 3 mm großen Schlitz somit für das Einführen der Kanüle und der Betätigung des radialen Kolbens lediglich einen Kolbenhub von etwa 10 mm. Die auf Kleinstmaß gerollte IOL tritt aus der Spitze der Kanüle aus, entrollt und wird mit Hilfe der Haptik in der Linsenkapsel des Auges oder gegebenenfalls an anderen vorgesehenen Orten positioniert.

Nachfolgend wird die Erfindung anhand von Schnittzeichnungen in Form eines Ausführungsbeispiels erläutert.
**Fig. 1** zeigt das Implantationswerkzeug mit der angeflanschten Kanüle und der hierin eingebetteten IOL in der Ausgangslage.
**Fig. 2** zeigt das Implantationswerkzeug nach der manuellen axialen Betätigung bis zum rückwärtigen Anschlag und die Position der IOL vor dem Austritt aus der Kanüle.
**Fig. 3** zeigt das Implantationswerkzeug nach der Betätigung des radial wirkenden Kolbens in der Endposition und die ausgetretene IOL.
**Fig. 4** zeigt die Kanüle in vereinfachter Darstellung in der Ausgangsposition mit dem an die IOL anliegenden Stößel.
**Fig. 5-11** zeigen die Durchtrittssituationen der IOL mit ihrer Haptik durch den Rollgang der Kanüle vom Transportbeginn bis zum Austrittsbeginn.
**Fig.12** zeigt das Implantationswerkzeug mit einer T-Führung zur Aufnahme der Kanüle.
**Fig.13** zeigt den Querschnitt der Kanüle im Bereich der T-Führung mit seitlichen Gleitschienen.
**Fig. 14** zeigt den Querschnitt des Implantationswerkzeugs im Bereich der T-Führung zur Aufnahme der Kanüle.
**Fig. 15-18** zeigen das Implantationswerkzeug mit-einem-Gleitmittelbehältnis in verschiedenen Einsatzphasen.

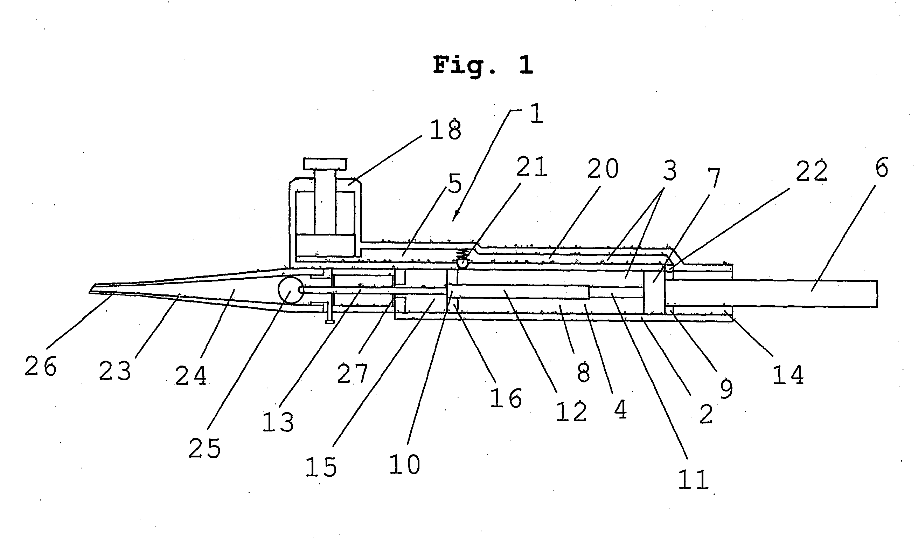

In **Fig. 1** ist ein Implantationswerkzeug 1 mit angeflanschter Kanüle 23 und der hierin eingebetteten IOL 25 erkennbar. Die Kanüle 23 bildet vorzugsweise sogleich das Implantierwerkzeug. Das Implantierwerkzeug kann jedoch auch mittels eines Adapters oder dergleichen an dem Implantationswerkzeug 1 befestigbar sein. Das Implantationswerkzeug 1 besteht aus einem Grundkörper 2, der eine Zweistufenhydraulik 3 beinhaltet, die sich zusammensetzt aus der ersten Hydraulikstufe 4 und der zweiten Hydraulikstufe 5. In dem röhrchenförmigen Grundkörper 2 ist eine Kolbenstange 6 mit Kolben 7 und einem dreistufigen Stößel 10 geführt, der aus einem kolbennahen Stößelabschnitt 11; einem mittleren Stößelabschnitt 12 und einem vorderen Stößelabschnitt 13 besteht. Der kolbennahe Stößelabschnitt 11 des Stößels 10 weist einen deutlich geringeren Durchmesser auf als die Kolbenstange 6. Der mittlere Stößelabschnitt 12 des Stößels 10 wiederum hat einen größeren Durchmesser als der Stößelabschnitt 11. Der vordere Stößelabschnitt 13 des Stößels 10 weist hingegen den geringsten Durchmesser auf und besteht zumindest in seiner Spitze aus einem elastischen Material, das einen schonenden Transport der IOL 25 gewährleistet. Der Grundköper 2 weist in seinem Endbereich eine Führungsbuchse 14 auf, die für die axiale Betätigung der Kolbenstange 6 einen deren Ausgangslage festlegenden Anschlag bildet.

Die Hydraulikstufe 4 weist zu beiden Seiten des Kolbens 7 jeweils einen Kolbenringraum 8 und 9 auf. Der Kolbenringraum 8 ist über ein entsperrbares Rückschlagventil 21 mit der Hydraulikstufe 5 und der Kolbenringraum 9 über eine Bohrung 22 mit der Hydraulikkammer 20 verbunden. Die Hydraulikkammer 20 ist mit dem Kolbenraum des Kolbens 17 in dem Zylinder 18 verbunden.

Der Kolbenringraum 8 ist durch eine Trennwand 16 von der Auffangkammer 15 getrennt. Bei der Verbindung der Kanüle 23 mit dem Grundkörper 2 wird die Folie 27 von der Spitze des vorderen Stößelabschnitts 13 des Stößels 10 durchdrungen und die Vorschubverbindung des Stößels 10 mit der IOL 25 hergestellt. Alternativ ist es auch möglich, den vorderen Stößelabschnitt 13 der Kolbenstange 6 als separates Bauteil auszulegen und vormontiert mit der Kanüle 23 und der hierin gelagerten IOL 25 anzuliefern. Die Kanüle 23 ist im Bereich der Kanülenspitze mit einer Rasteinziehung versehen, in die eine Rasterhöhung einer Verschlusskappe 26 zur Verbindung der beiden Teile einrastet.

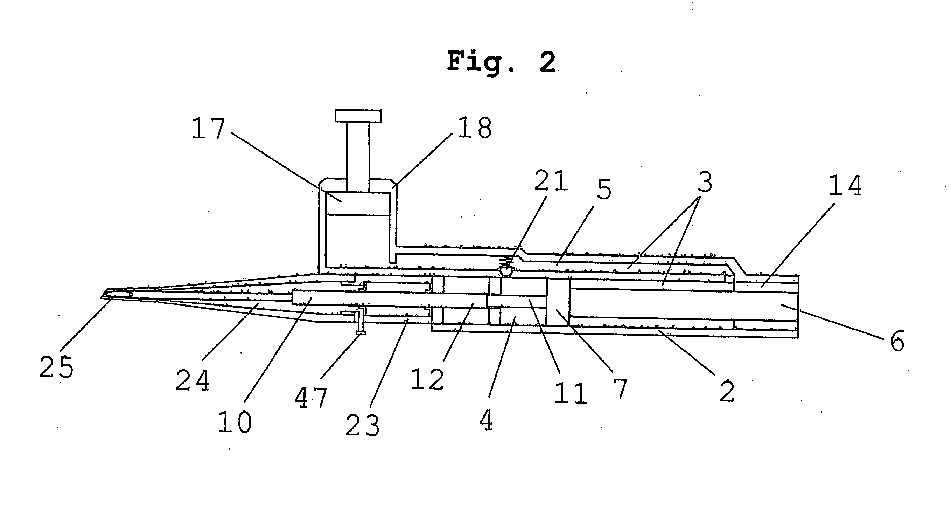

Die **Fig. 2** zeigt das Implantationswerkzeug mit etwa dreiviertel ausgefahrenem Kolben 7. Diese Kolbenstellung wird begrenzt durch die Führungsbuchse 14, die den Hub der ersten Hydraulikstufe 4 definiert. Die Kolbenstange 6 wird hierzu soweit axial verschoben, bis sie plan mit der Außenfläche der Führungsbuchse 14 ist. Durch die Vorschubbewegung des Kolbens 7 wird die Hydraulikflüssigkeit von der ersten Hydraulikstufe 4 über das Rückschlagventil 21 in die Hydraulikkammer 20 und den Kolbenringraum 9 gedrückt. Der Kolbenringraum 8 wird bei dieser Aktion bis auf etwa einviertel seines Volumens eingefahren und der Kolben 17 bis zum Deckel des Zylinders 18 ausgefahren. Der kolbennahe Stößelabschnitt des Stößels 10 gelangt gleichzeitig in eine Situation, in der die Trennwand 16 von dem Ende des mittleren Stößelabschnitts 12 des Stößels 10 verlassen wird. Die IOL 25 ist durch den Rollgang 24 der Kanüle 23 bis zum Austritt in den Kapselsack des Auges transportiert worden.

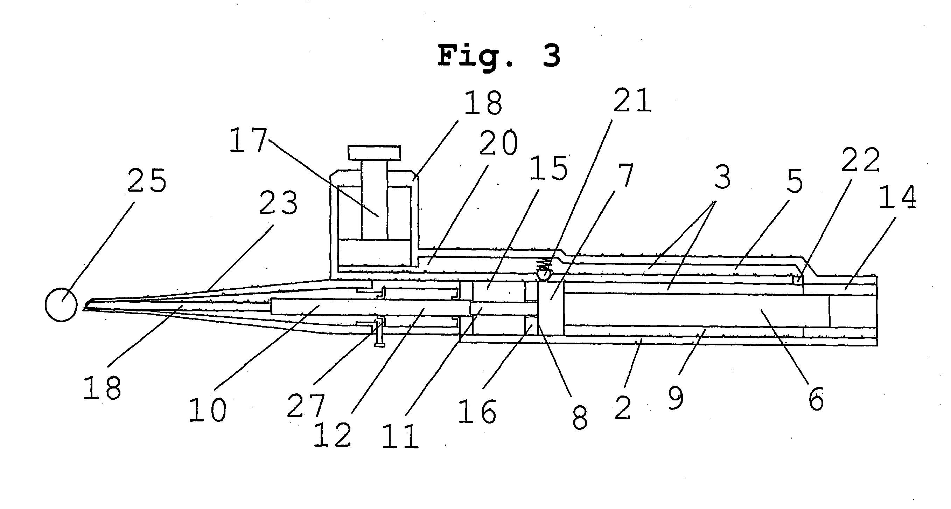

Die **Fig. 3** zeigt den Austritt der IOL 25 aus der Kanüle 23 nach Beendigung des Vorschubs des Kolbens 7. Der Vorschub des Kolbens 7 bis zum Anschlag an die Trennwand 16 wird durch Betätigen der zweiten Hydraulikstufe 5 vollzogen. Hierzu wird nun der radial verfahrbare Kolben 17 eingefahren, die verbliebene Hydraulikflüssigkeit des Kolbenringraums 8 durch die geöffnete Trennwand 16 in die mit einem Atmungsfilter ausgerüstete Auffangkammer 15 gedrückt und gleichzeitig der Kolbenringraum 9 mit der vom Kolben 17 verdrängten Hydraulikflüssigkeit beaufschlagt.

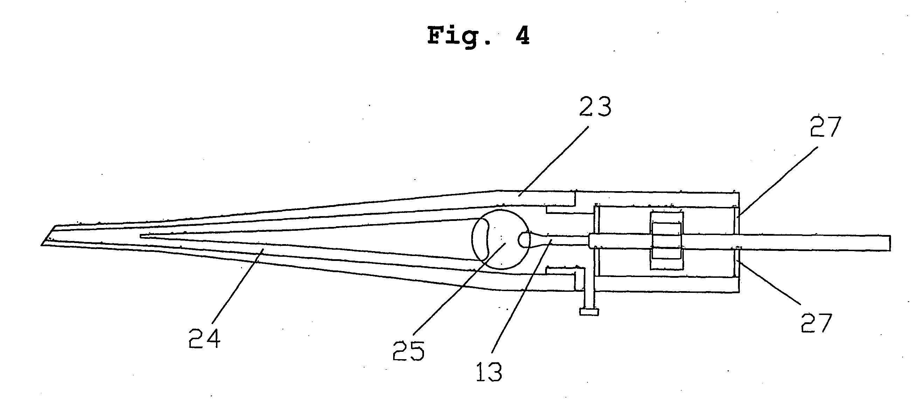

**Fig. 4** zeigt die Kanüle 23 losgelöst vom Grundkörper 2 des Implantationswerkzeugs 1. Es ist die eingelagerte IOL 25 ohne Haptik dargestellt erkennbar. Der elastische vordere Stößelabschnitt 13 des Stößels 10 hat die Folie 27 durchstoßen und ist vorschubbereit mit der IOL 25 in Kontakt. Der Rollgang 24 ist zum Transport mit Gleitmittel gefüllt und der Vorschub der IOL 25 kann beginnen.

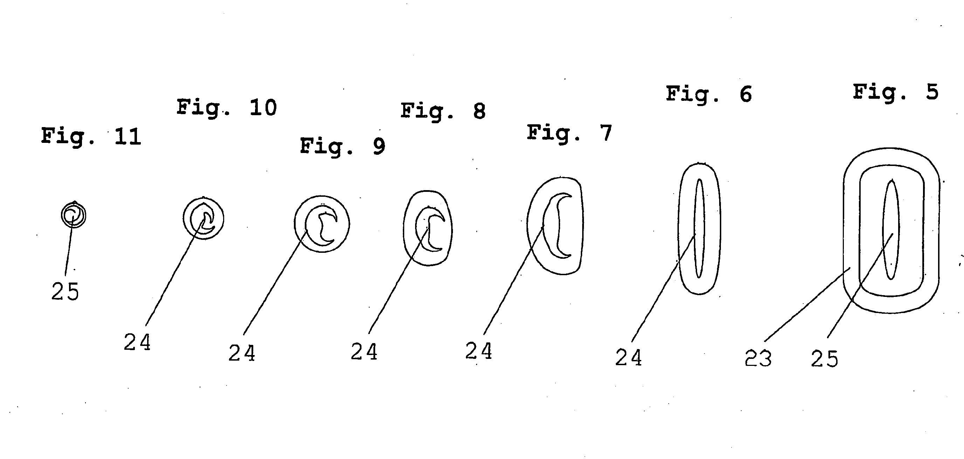

Die **Fig. 5 -11** zeigen die einzelnen Transportsituationen der IOL 25 von der Aufbewahrungsphase bis zum Austritt aus dem Rollgang 24 der Kanüle 23.

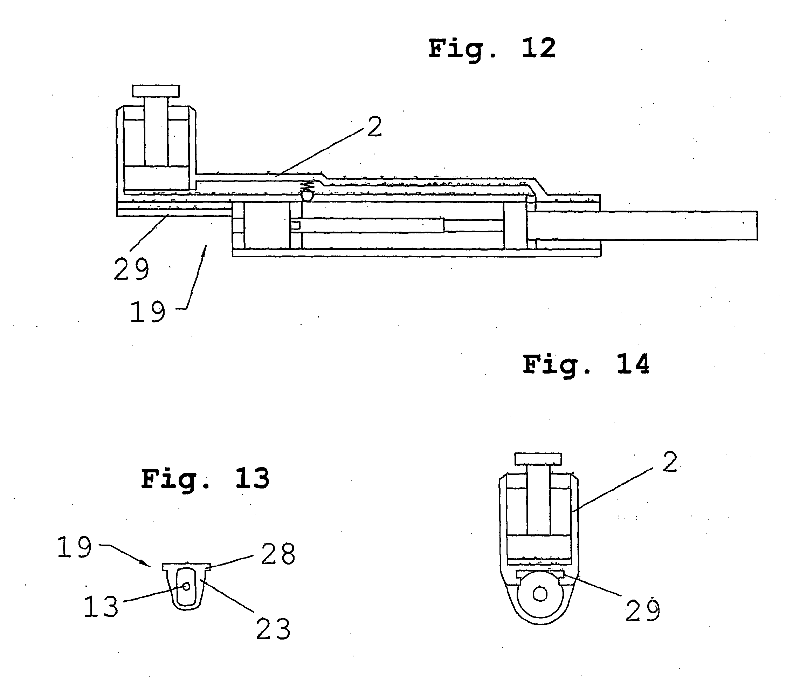

Die **Fig.12-14** zeigen eine Verbindungsmöglichkeit der Kanüle 23 mit dem Implantationswerkzeug 2 mittels einer T-Führung 29 und hierin eingreifender seitlicher Gleitschienen 28.

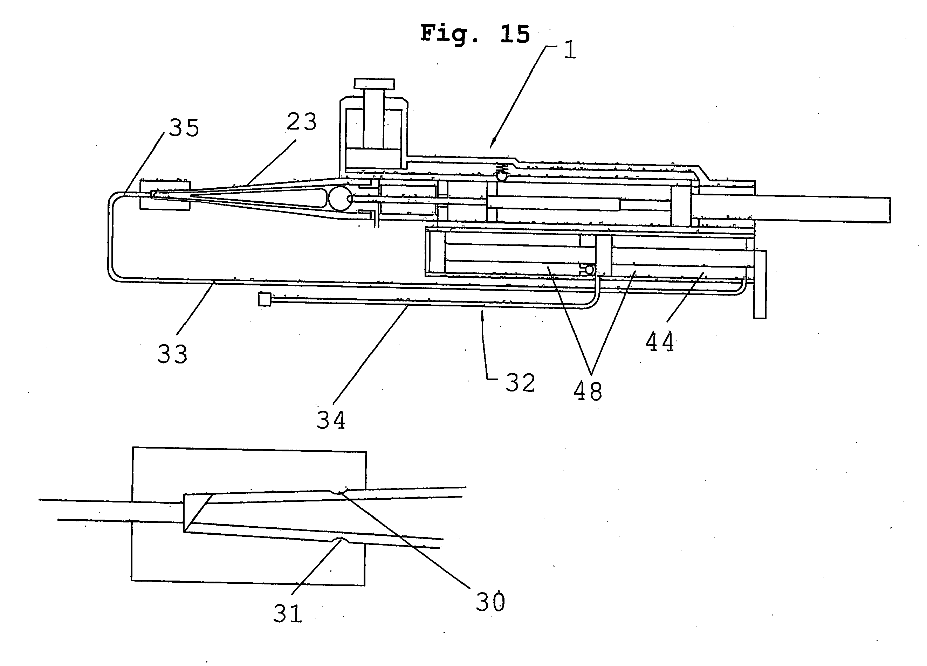

**Fig. 15** zeigt das Implantationswerkzeug 1 mit formschlüssig verbundenem Gleitmittelbehältnis 32. Der Befüllungsschlauch 33 ist auf die Injektorspitze der Kanüle 23 aufgesetzt und mittels Klemme 35 verschlossen.

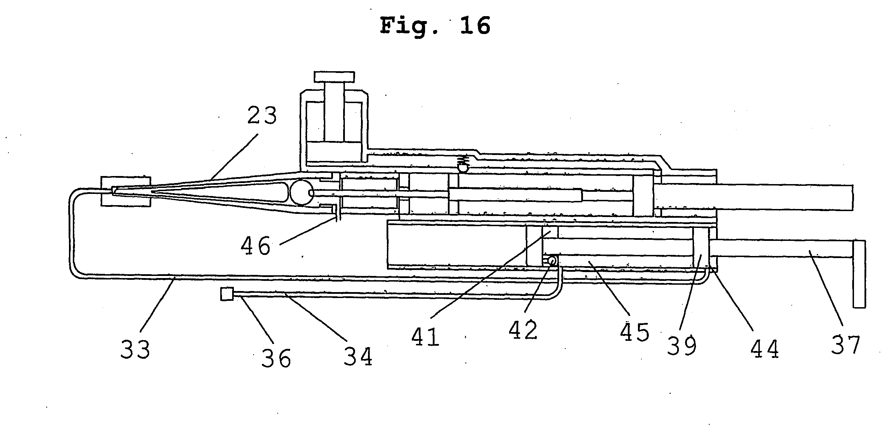

In **Fig.16** ist der Stopfen 47 vom Ausgangsstutzen 46 abgezogen, die Klemme 35 entfernt und die Kolbenstange 37 des Gleitmittelbehältnisses 32 ausgezogen worden. Hierbei wird der Kolbenringraum 44 über den Befüllungsschlauch 33 in die Kanüle 23 entleert und gleichzeitig das Volumen des Kolbenringraumes 45 durch die Bohrung 42 und über das Rückschlagventil 43 durch die Zylinderzwischenwand 41 in den anderen Kolbenringraum 45' gedrückt. Das so umgepumpte Gleitmittel 48 verdrängt die sterile Aufbewahrungsflüssigkeit durch den Ausgangsstutzen 46 der Kanüle 23.

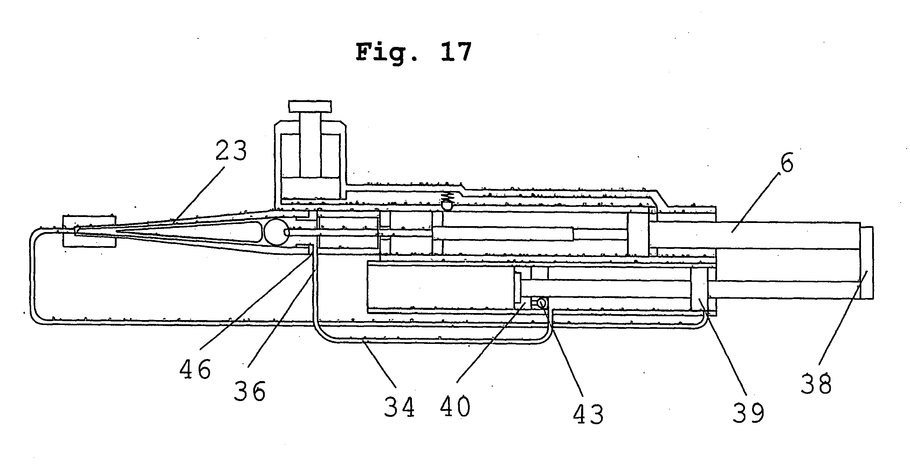

In **Fig. 17** ist erkennbar, dass das abgewinkelte Ende 38 der Kolbenstange 37 hochgeschwenkt und in Anlage mit dem Ende der Kolbenstange 6 -gebracht wird. Anschließend wird der Befüllungsschlauch 34 am hinteren Teil der Kanüle 23 mit dem Ausgangsstutzen 46 verbunden und die Klemme 36 abgezogen.

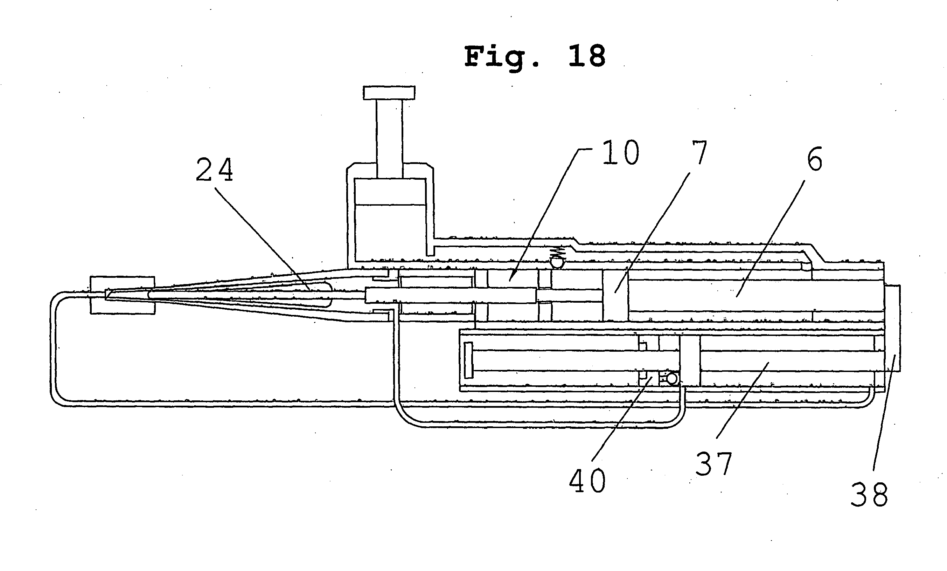

**Fig.18** zeigt die wieder eingefahrene Kolbenstange 37, die über ihr abgewinkeltes Ende 38 die Kolbenstange 6 bis zum Anschlag an die Führungsbuchse 14 mitgenommen hat. Hierbei wird über den Befüllungsschlauch 33 Gleitmittel 48 abgesaugt und gleichzeitig über den Befiillungsschlauch 34 Gleitmittel 48 durch den Ausgangsstutzen 46 hinter der IOL 25 unter Druck eingebracht. Bei diesem Schubvorgang entsteht vor der IOL 25 ein hydrostatischer Unterdruck und hinter der IOL 25 ein hydrostatischer Überdruck. Die IOL 25 wird somit durch den Rollgang 24 nur mittels geringer statischer Druckbelastung über die Kolbenstange 6 und den Kolben 7 sowie dem dreistufigen Stößel 10 in ihre gerollte Austrittsposition befördert. Damit dieser Arbeitsgang reibungslos durchgeführt werden kann, ist der Kolben als loser Kolben 40 auf der Kolbenstange 7 verschiebbar angeordnet.

Nach diesem Arbeitsgang werden die Befüllungsschläuche 33, 34 abgezogen, der Ausgangsstutzen 46 durch den Stopfen 47 verschlossen und das Gleitmittelbehältnis 32 vom Implantationswerkzeug 1 demontiert. Das soweit vom medizinischen Personal vorbereitete Implantationswerkzeug 1 entspricht nun wieder der Fig. 2 und wird dem Operateur für den letzten Arbeitsgang übergeben.

Es versteht sich, dass die Erfindung auf das vorstehend beschriebene Ausführungsbeispiel nicht beschränkt ist, dieses in Einzelheiten Änderungen erfahren kann, ohne den Rahmen der Erfindung zu verlassen.

### Bezugszeichenliste

- 1: Implantationswerkzeug
- 2: Grundkörper
- 3: Zweistufenhydraulik
- 4: Erste Hydraulikstufe
- 5: Zweite Hydraulikstufe
- 6: Kolbenstange
- 7: Kolben
- 8: Kolbenringraum
- 9: Kolbenringraum
- 10: Dreistufiger Stößel
- 11: Kolbennaher Stößelabschnitt des Stößels
- 12: Mittlerer Stößelabschnitt des Stößels
- 13: Vorderer Stößelabschnitt des Stößels
- 14: Führungsbuchse
- 15: Auffangkammer mit Atmungsfilter
- 16: Trennwand
- 17: Kolben
- 18: Zylinder
- 19: Verbindung
- 20: Hydraulikkammer
- 21: Rückschlagventil
- 22: Bohrung
- 23: Kanüle
- 24: Rollgang
- 25: IOL
- 26: Verschlusskappe
- 27: Folie
- 28: Gleitschiene
- 29: T-Führung
- 30: Rasteinziehung
- 31: Rasterhöhung
- 32: Gleitmittelbehältnis
- 33: Befüllungsschlauch
- 34: Befüllungsschlauch
- 35: Klemme
- 36: Klemme
- 37: Kolbenstange
- 38: abgewinkeltes Ende der Kolbenstange
- 39: Kolben
- 40: loser Kolben
- 41: Zylinderzwischenwand
- 42: Bohrung
- 43: Rückschlagventil
- 44: Kolbenringraum
- 45: Kolbenringraum
- 45': Kolbenringraum
- 46: Ausgangsstutzen
- 47: Stopfen
- 48: Gleitmittel

## Patentansprüche

1. Chirurgisches Implantationswerkzeug (1) zum Implantieren einer verformbaren Intraokularlinse (25) aus weichem faltbaren Material, insbesondere Silikon oder Acryl, in ein Auge, mit einem röhrchenförmigen Grundkörper (2), vorzugsweise mit einem röhrchenförmigen, im Innendurchmesser an die Linse angepassten Implantierwerkzeug, mit dem die Linse durch eine Schnittöffnung im Auge in die Linsenkapsel des Auges einsetzbar ist, mit einem Stößel (10) und mit einer Schubeinrichtung zum Herausschieben der Linse aus dem Implantierwerkzeug mittels des Stößels, **dadurch gekennzeichnet, dass** die Schubeinrichtung als Antrieb für den Stößel mit einer Zweistufenhydraulik **(3)** versehen ist.

2. Implantationswerkzeug nach Anspruch 1, **dadurch gekennzeichnet, dass** die Schubeinrichtung aus einer rückwärtigen Kolbenstange **(6)** und einem etwa mittig angeordneten Kolben **(7)** besteht, dem der Stößel **(10)** vorgeschaltet ist.

3. Implantationswerkzeug nach Anspruch 1 oder 2, **dadurch gekennzeichnet, dass** der Stößel **(10)** dreistufig ausgelegt ist.

4. Implantationswerkzeug nach Anspruch 3; **dadurch gekennzeichnet, dass** der kolbennahe Stößelabschnitt **(11)** des Stößels **(10)** einen geringeren Durchmesser aufweist, als der mittlere Stößelabschnitt **(12)** des Stößels **(10)** und der vordere Stößelabschnitt **(13)** einen geringeren Durchmesser aufweist als der kolbennahe Stößelabschnitt **(11).**

5. Implantationswerkzeug nach Anspruch 4, **dadurch gekennzeichnet, dass** der vordere Stößelabschnitt **(13)** des Stößels **(10)** separat vom Stößel in einer vorzugsweise das Implantierwerkzeug bildenden Kanüle **(23)** verschiebbar gelagert ist.

6. Implantationswerkzeug nach den Ansprüchen 4 und 5, **dadurch gekennzeichnet, dass** vorzugsweise der vordere Stößelabschnitt **(13)** des Stößels **(10)** aus flexiblem Material besteht.

7. Implantationswerkzeug nach einem der Anspräche 1 bis 6, **dadurch gekennzeichnet, dass** die Schubeinrichtung in dem die erste Hydraulikstufe **(4)** bildenden röhrchenförmigen Grundkörper **(2)** geführt ist.

8. Implantationswerkzeug nach einem der Ansprüche 1 bis 7, **dadurch gekennzeichnet, dass** die erste Hydraulikstufe **(4)** im Grundkörper **(2)** im rückwärtigen Ende eine verhältnismäßig lange Führungsbuchse **(14)** für die Kolbenstange **(6)** aufweist.

9. Implantationswerkzeug nach einem der Ansprüche 1 bis 8, **dadurch gekennzeichnet, dass** der ersten Hydraulikstufe **(4)** im Grundkörper (2) eine Auffangkammer **(15)** mit Atmungsfilter vorgeschaltet ist.

10. Implantationswerkzeug nach einem der Ansprüche 1 bis 9, **dadurch gekennzeichnet, dass** radial auf dem Grundkörper **(2)** ein radial zu betätigender Kolben **(17)** mit der zweiten Hydraulikstufe **(5)** aufgesattelt ist.

11. Implantationswerkzeug nach einem der Ansprüche 1 bis 10, **dadurch gekennzeichnet, dass** die erste Hydraulikstufe **(4)** durch ein Rückschlagventil **(21)** gegen die zweite Hydraulikstufe **(5)** abgesichert ist.

12. Implantationswerkzeug nach einem der Ansprüche 1 bis 11, **dadurch gekennzeichnet, dass** die erste Hydraulikstufe (4) durch eine Bohrung **(22)** mit der zweiten Hydraulikstufe **(5)** verbunden ist.

13. Implantationswerkzeug nach Anspruch 7, **dadurch gekennzeichnet, dass** der Grundkörper **(2)** derart ausgestaltet ist, dass an diesem durch eine einfache, lösbare Verbindung **(19)** eine Kanüle **(23)** befestigbar oder befestigt ist.

14. Implantationswerkzeug nach Anspruch 13, **dadurch gekennzeichnet, dass** in der Kanüle **(23)** die zu implantierende Intraokularlinse **(25)** positioniert oder positionierbar ist.

15. Implantationswerkzeug nach den Ansprüchen 13 und 14, **dadurch gekennzeichnet, dass** die Kanüle **(23)** einen Rollgang **(24)** aufweist.

16. Implantationswerkzeug nach Anspruch 15, **dadurch gekennzeichnet, dass** sich der Rollgang **(24)** düsenartig verjüngt.

17. Implantationswerkzeug nach Anspruch 13 bis 16, **dadurch gekennzeichnet, dass** vor dem Ausgangsbereich des Rollgangs **(24)** eine abziehbare oder abtrennbare Verschlusskappe **(26)** vorgesehen ist.

18. Implantationswerkzeug nach Anspruch 13 bis 17 **dadurch gekennzeichnet, dass** der rückwärtige Bereich der Kanüle **(23)** durch eine leicht zu durchstoßende Folie **(27)** verschlossen ist.

19. Implantationswerkzeug nach Anspruch 13 bis 18 **dadurch gekennzeichnet, dass** der Innenraum der Kanüle **(23)** mit vorzugsweise destilliertem Wasser gefüllt wird.

20. Implantationswerkzeug nach Anspruch 17, **dadurch gekennzeichnet, dass** durch die zentrale Öffnung der Kanüle **(23)** Gleitmittel eingeführt werden kann.

21. Implantationswerkzeug nach Anspruch 1 bis 20, **dadurch gekennzeichnet, dass** der Grundkörper **(2)** aus in einem Autoklaven sterilisierbaren medizinischem Kunststoff, nicht rostendem Stahl oder vorzugsweise Titan bzw. einer Titanlegierung hergestellt wird.

22. Implantationswerkzeug nach Anspruch 21, **dadurch gekennzeichnet, dass** der Grundkörper **(2)** nach der Sterilisierung wieder verwertbar ist.

23. Implantationswerkzeug nach Anspruch 2, **dadurch gekennzeichnet, dass** durch ein Einfahren der rückwärtigen Kolbenstange **(7)** bis zum endseitigen Anschlag ein Teilbereich der ersten Hydraulikstufe **(4)** begrenzt und die zweite Hydraulikstufe **(5)** mit der Hydraulikflüssigkeit dieses Teilbereichs der ersten Hydraulikstufe **(4)** gefüllt wird.

24. Implantationswerkzeug nach Anspruch 23, **dadurch gekennzeichnet, dass** im Übergangsbereich von der ersten zur zweiten Hydraulikstufe die Verbindung zur Auffangkammer **(15)** aufgefahren wird.

25. Implantationswerkzeug nach einem der Ansprüche 22 und 23, **dadurch gekennzeichnet, dass** die zweite Hydraulikstufe **(5)** durch den radial zu betätigenden Kolben **(17)** beaufschlagt, der Kolbenringraum **(9)** von der zweiten Hydraulikstufe **(5)** gefüllt und der Kolben **(7)** gegen den vorderen Endanschlag gefahren wird.

26. Implantationswerkzeug nach Anspruch 25, **dadurch gekennzeichnet, dass** der radial wirkende Kolben **(17)** ausgefahren, danach das entsperrbare Rückschlagventil **(21)** geöffnet, der Kolben **(17)** wieder eingefahren und **dadurch** die Zweistufenhydraulik **(3)** in ihre Ausgangslage gebraucht wird.

27. Implantationswerkzeug nach einem der Ansprüche 1 bis 26, **dadurch gekennzeichnet, dass** mit dem Implantationswerkzeug **(1)** ein Gleitmittelbehältnis **(32)** lösbar verbunden ist.

28. Implantationswerkzeug mit Gleitmittelbehältnis nach Anspruch 27, **dadurch gekennzeichnet, dass** das Gleitmittelbehältnis **(32)** mit der Kanüle **(23)** über zwei Befüllungsschläuche **(33, 34)** verbunden ist.

29. Implantationswerkzeug mit Gleitmittelbehältnis nach Anspruch 28, **dadurch gekennzeichnet, dass** der Befüllungsschlauch **(33)** mit der Spitze der Kanüle **(23)** und der Befüllungsschlauch **(34)** über einen Ausgangsstutzen **(46)** mit dem Kanülenende verbunden ist.

30. Gleitmittelbehältnis für ein Implantationswerkzeug nach Anspruch 27, **dadurch gekennzeichnet, dass** das Gleitmittelbehältnis **(32)** aus einem einseitig offenen Zylinder besteht.

31. Gleitmittelbehältnis nach Anspruch 30, **dadurch gekennzeichnet, dass** die Kolbenstange **(37)** zweiteilig ist und mittig einen Kolben **(39)** und einenends einen losen Kolben **(40)** aufweist.

32. Gleitmittelbehältnis nach Anspruch 31, **dadurch gekennzeichnet, dass** das ausfahrbare Kolbenstangenende ein um 90° abgewinkeltes Ende aufweist.

## Claims

1. Surgical implantation tool (1) for implantation of a deformable intraocular lens (25) made of soft foldable material, particularly silicone or acrylic into an eye, with a tubular main body (2), preferably with a tubular implant tool which has its internal diameter adapted to the lens, with which the lens can be inserted into the lens capsule of the eye through an incision in the eye and the implantation tool provided with a tappet (10) and with a pushing device for pushing the lens out of the implant tool by means of the tappet wherein the pushing device is provided with a two-stage hydraulic mechanism (3) as drive for the tappet.

2. Implantation tool according to claim 1 wherein the pushing device consists of a rearward piston rod (6), and a piston (7) arranged about in the center, to which the tappet (10) is connected upstream.

3. Implantation tool according to claims 1 or 2 wherein the tappet (10) is of three-stage design.

4. Implantation tool according to claim 3 wherein the piston-end tappet section (11) of the tappet (10) has a smaller diameter than the center tappet section (12) of the tappet (10) and the front tappet section (13) has a smaller diameter than the piston-end tappet section (11).

5. Implantation tool according to claim 4 wherein the front tappet section (13) of the tappet (10) is supported separately from the tappet and slidably in a cannula (23) preferably forming the implant tool.

6. Implantation tool according to claims 4 and 5 wherein the front tappet section (13) of the tappet (10) preferably consists of flexible material.

7. Implantation tool of any of the claims 1 to 6 wherein the pushing device is guided in a tubular main body (2) forming the first hydraulic stage (4).

8. Implantation tool of any of the claims 1 to 7 wherein the first hydraulic stage (4) in the main body (2) at the rearward end is provided with a relatively long guide bush (14) for the piston rod (6).

9. Implantation tool of any of the claims 1 to 8 wherein a collecting chamber (15) with breather filter is connected upstream of the first hydraulic stage (4) in the main body (2).

10. Implantation tool of any of the claims 1 to 9 wherein a piston (17) to be operated radially incorporating the second hydraulic stage (5) is mounted radially on the main body (2).

11. Implantation tool of any of the claims 1 to 10 wherein the first hydraulic stage (4) is protected from the second hydraulic stage (5) by a check valve (21).

12. Implantation tool of any of the claims 1 to 11 wherein the first hydraulic stage (4) is connected with the second hydraulic stage (5) via a hole (22).

13. Implantation tool according to claim 7 wherein the main body (2) is designed such that a cannula (23) is connectable or connected to it by means of a simple connection (19).

14. Implantation tool according to claim 13 wherein the intraocular lens (25) to be implanted is or can be positioned in the cannula (23).

15. Implantation tool according to claims 13 and 14 wherein the cannula (23) comes provided with a roller path (24).

16. Implantation tool according to claim 15 wherein the roller path (24) tapers like a nozzle.

17. Implantation tool according to claims 13 to 16 wherein a detachable or removable sealing cap (26) is provided before the exit area of the roller path (24).

18. Implantation tool according to claims 13 to 17 wherein the rearward area of the cannula (23) is closed by a film (27) which can be punctured easily.

19. Implantation tool according to claims 13 to 18 wherein the interior of the cannula (23) is preferably filled with distilled water.

20. Implantation tool according to claim 17 wherein a lubricant fluid can be introduced through the central opening of the cannula (23).

21. Implantation tool according to claims 1 to 20 wherein the main body (2) is made from medical plastics, stainless steel or preferably titanium or a titanium alloy sterilizable in an autoclave.

22. Implantation tool according to claim 21 wherein the main body (2) is reusable after sterilization.

23. Implantation tool according to claim 2 wherein a partial area of the first hydraulic stage (4) is limited by retracting the rearward piston rod (7) up to the end-side stop and the second hydraulic stage (5) is filled with the hydraulic fluid of said partial area of the first hydraulic stage (4).

24. Implantation tool according to claim 23 wherein the connection to the collecting chamber (15) is open in the transition area between the first and second hydraulic stages.

25. Implantation tool of any of the claims 22 and 23 wherein the second hydraulic stage (5) is being loaded by the piston (17) to be operated radially, the piston annulus (9) is filled from the second hydraulic stage (5) and the piston 87) is extended until it contacts the foremost end stop.

26. Implantation tool according to claim 25 wherein the radially acting piston (17) is extended, then the pilot check valve (21) is opened, the piston (17) retracted again thus returning the two-stage hydraulic mechanism (3) to its original position.

27. Implantation tool according to any of the claims 1 to 26 wherein a lubricant fluid container (32) is detachably connected with the implantation tool (1).

28. Implantation tool with lubricant fluid container according to claim 27 wherein the lubricant fluid container (32) is connected to the cannula (23) via two filling hoses (33, 34).

29. Implantation tool with lubricant fluid container according to claim 28 wherein the filling hose (33) is connected to the tip of the cannula (23) and the filling hose (34) is connected via an outlet socket (46) to the end of the cannula.

30. Lubricant fluid container for an implantation tool according to claim 27 wherein the lubricant fluid container (32) consists of a cylinder being open at one end.

31. Lubricant fluid container according to claim 30 wherein the piston rod (37) is bipartite and has a piston (39) in the center and a loose piston (40) at one end.

32. Lubricant fluid container according to claim 31 wherein the extendable piston rod end is provided with an end angled by 90°.

## Revendications

1. Outil d'implantation (1) chirurgical, pour implanter dans un oeil une lentille intraoculaire (25) déformable en matériau pliable souple, en particulier du silicone ou de l'acryle, comprenant un corps de base (2) en forme de petit tuyau, de préférence comprenant un outil pour implanter en forme de petit tuyau, adapté à la lentille en diamètre intérieur, avec lequel la lentille peut être introduite dans la capsule du cristallin de l'oeil à travers une incision dans l'oeil, comprenant un poussoir (10) et comprenant un dispositif de poussée pour pousser au moyen du poussoir la lentille en dehors de l'outil pour implanter, **caractérisé en ce que** le dispositif de poussée est muni d'une hydraulique à deux étages (3) en tant qu'entraînement pour le poussoir.

2. Outil d'implantation selon la revendication 1, **caractérisé en ce que** le dispositif de poussée consiste en une tige de piston (6) arrière et un piston (7) agencé environ au milieu, en amont duquel est placé le poussoir (10).

3. Outil d'implantation selon la revendication 1 ou 2, **caractérisé en ce que** le poussoir (10) est arrangé pour avoir trois étagements.

4. Outil d'implantation selon la revendication 3, **caractérisé en ce que** le tronçon (10) du poussoir (11) qui est proche du piston présente un diamètre inférieur au tronçon médian (12) du poussoir (10) et le tronçon avant (13) du poussoir présente un diamètre inférieur au tronçon (11) proche du piston.

5. Outil d'implantation selon la revendication 4, **caractérisé en ce que** le tronçon avant (13) du poussoir (10) est monté déplaçable, séparément du poussoir, dans une canule (23) formant de préférence l'outil pour implanter.

6. Outil d'implantation selon les revendications 4 et 5, **caractérisé en ce que** de préférence, le tronçon avant (13) du poussoir (10) est en matériau flexible.

7. Outil d'implantation selon l'une des revendications 1 à 6, **caractérisé en ce que** le dispositif de poussée est guidé dans le corps de base (2) en forme de petit tuyau formant le premier étage hydraulique (4).

8. Outil d'implantation selon l'une des revendications 1 à 7, **caractérisé en ce que** le premier étage hydraulique (4) présente dans le corps de base (2) dans l'extrémité arrière une douille de guidage (14) relativement longue pour la tige de piston (6).

9. Outil d'implantation selon l'une des revendications 1 à 8, **caractérisé en ce qu'**une chambre collectrice (15) comprenant un filtre de respiration est située en amont du premier étage hydraulique (4) dans le corps de base (2).

10. Outil d'implantation selon l'une des revendications 1 à 9, **caractérisé en ce qu'**un piston (17) à actionner radialement est installé radialement sur le corps de base (2) avec le deuxième étage hydraulique (5).

11. Outil d'implantation selon l'une des revendications 1 à 10, **caractérisé en ce que** le premier étage hydraulique (4) est protégé du deuxième étage hydraulique (5) via un clapet anti-retour (21).

12. Outil d'implantation selon l'une des revendications 1 à 11, **caractérisé en ce que** le premier étage hydraulique (4) est relié au deuxième étage hydraulique (5) par un perçage (22).

13. Outil d'implantation selon la revendication 7, **caractérisé en ce que** le corps de base (2) est arrangé de façon qu'une canule (23) puisse être ou soit fixée à celui-ci via une liaison (19) amovible simple.

14. Outil d'implantation selon la revendication 13, **caractérisé en ce que** la lentille intraoculaire (25) à implanter peut être ou est positionnée dans la canule (23).

15. Outil d'implantation selon les revendications 13 et 14, **caractérisé en ce que** la canule (23) présente un passage d'enroulement (24).

16. Outil d'implantation selon la revendication 15, **caractérisé en ce que** le passage d'enroulement (24) se rétrécit à la façon d'une tuyère.

17. Outil d'implantation selon la revendication 13 à 16, **caractérisé en ce qu'**il est prévu devant la région de sortie du passage d'enroulement (24) un bouchon (26) pouvant être détaché ou extrait.

18. Outil d'implantation selon la revendication 13 à 17, **caractérisé en ce que** la région arrière de la canule (23) est fermée par un film (27) facilement transperçable.

19. Outil d'implantation selon la revendication 13 à 18, **caractérisé en ce que** l'espace intérieur de la canule (23) est rempli avec de préférence de l'eau distillée.

20. Outil d'implantation selon la revendication 17, **caractérisé en ce qu'**un lubrifiant peut être introduit à travers l'ouverture centrale de la canule (23).

21. Outil d'implantation selon la revendication 1 à 20, **caractérisé en ce que** le corps de base (20) est fabriqué à partir d'une matière synthétique médicale pouvant être stérilisée dans un autoclave, de l'acier inoxydable ou de préférence du titane ou un alliage de titane.

22. Outil d'implantation selon la revendication 22, **caractérisé en ce que** le corps de base (2) est réutilisable après stérilisation.

23. Outil d'implantation selon la revendication 2, **caractérisé en ce qu'**une région partielle du premier étage hydraulique (4) est délimitée par une entrée de la tige de piston (7) arrière jusqu'à une butée d'extrémité, et le deuxième étage hydraulique (5) est rempli avec le fluide hydraulique de cette région partielle du premier étage hydraulique (4).

24. Outil d'implantation selon la revendication 23, **caractérisé en ce que** la liaison vers la chambre collectrice (15) est atteinte dans la région de la transition du premier au deuxième étage hydraulique.

25. Outil d'implantation selon la revendication 22 ou 23, **caractérisé en ce que** le deuxième étage hydraulique (5) est alimenté par le piston (17) à actionner radialement, la chambre annulaire de piston (9) du deuxième étage hydraulique (5) est remplie et le piston (7) est déplacé contre la butée d'extrémité avant.

26. Outil d'implantation selon la revendication 25, **caractérisé en ce que** le piston (17) agissant radialement est sorti, ensuite clapet anti-retour (21) pouvant être débloqué est ouvert, le piston (17) repoussé vers l'intérieur et ainsi l'hydraulique à deux étages (3) est ramenée dans sa situation initiale.

27. Outil d'implantation selon l'une des revendications 1 à 26, **caractérisé en ce qu'**un récipient pour lubrifiant (32) est relié de façon amovible à l'outil d'implantation (1).

28. Outil d'implantation comprenant un récipient pour lubrifiant selon la revendication 27, **caractérisé en ce que** le récipient pour lubrifiant (32) est relié à la canule (23) via deux tuyaux de remplissage (33, 34).

29. Outil d'implantation comprenant un récipient pour lubrifiant selon la revendication 28, **caractérisé en ce que** le tuyau de remplissage (33) est relié à la pointe de la canule (23) et le tuyau de remplissage (34) est relié à l'extrémité de la canule via un raccord de sortie (46).

30. Récipient pour lubrifiant pour un outil d'implantation selon la revendication 27, **caractérisé en ce que** le récipient pour lubrifiant (32) consiste en un cylindre ouvert d'un côté.

31. Récipient pour lubrifiant selon la revendication 30, **caractérisé en ce que** la tige de piston (37) est en deux parties et présente au milieu un piston (39) et à une extrémité un piston (40) libre.

32. Récipient pour lubrifiant selon la revendication 31, **caractérisé en ce que** l'extrémité de tige de piston pouvant être sortie présente une extrémité coudée à 90°.
